# EUROPEAN PATENT APPLICATION

(11) **EP 2 757 076 A1**
(43) Date of publication of application: **23.07.2014**
(21) Application number: 14151869.6
(22) Date of filing: 21.01.2014
(51) Int. Cl.: C02F 9/00, C02F 101/16, C02F 1/66, C02F 103/20, C02F 11/00, C02F 1/20

(54) **Process for nitrogen extraction from digestates obtained by metanogenic fermentation, zootechnical dejecta and sewage sludges**

(30) Priority: 22.01.2013 IT PC20130004
(71) Applicant: Syngen S.r.l., 29122 Piacenza (IT)
(72) Inventor: CELLA, Fabio, 29122 Piacenza (IT)
(74) Representative: Raimondi, Margherita

(57) **Abstract**

Process for elimination of nitrogen from biomass DIG0 and reuse thereof for energy purposes, comprising the following steps:
A) alkaline conditioning of the biological starting material DIG0, adding calcium oxide (CaO) thereto and forming a first biomass DIG1 with pH>10 and free ammonia (NH₃) in gaseous form;
B) stripping of the ammonia (NH₃) from said first DIG1 biomass resulting from step A) via injection of air, with generation of a second biomass DIG2, free from ammonia, with low nitrogen content and alkaline pH, and a gaseous fluid GAS1 containing air, ammonia (NH₃) and water vapour;
C) introduction of the gaseous fluid GAS1 resulting from step B) into an internal combustion cogenerator, fuelled by biogas or other fuel, with use of the ammonia (NH₃) contained in the GAS1 for energy purposes, by means of combustion thereof together with the main fuel, and reduction of the nitrogen oxides present in the exhaust gases (GAS2) of the cogenerator by means of catalytic converters;
D) purification, from nitrogen oxides, of the gaseous fluid GAS2 containing NOx, H₂O, CO₂ and N₂ generated during step C) by means of:
- injection of the gaseous fluid GAS2 into the said biomass DIG2 resulting from step B);
- combination of the nitrogen oxides (NOx) of the gaseous fluid GAS2 with the alkaline mass of the biomass DIG2 with formation of calcium nitrite [Ca(NO₂)₂] and calcium nitrate [Ca(NO₃)₂] and simultaneous lowering of the pH of the biomass DIG2;
- production of a gaseous fluid GAS3 devoid of NOx and a resultant biomass DIG3 with pH lower than the pH of DIG2.

## Description

The present invention relates to a process for extraction and recovery for energy use of the nitrogen contained in biomass such as livestock manure, biological sewage sludge and residual biomass.

It is known in the technical sector relating to the treatment of agricultural soils that a vast range of biological materials, such as digestates from methanogenic fermentation, livestock manure, biological sewage sludge and residual biomass, may, if suitably treated, provided a valuable contribution for soil correction and the nutrition of plants since, in addition to the organic substance, these materials also contain considerable quantities of nitrogen, phosphorus, potassium, calcium and sulphur.

The said biological materials also include materials which are loose and soft, but have a dry substance content of more than 50%, such as poultry droppings from free-range farms.

On average the digestates from methanogenic fermentation resulting from the treatment of biological materials performed using liquid technologies contain a percentage of dry substance of between 1% and 10% which depends on the type of starting materials which undergo anaerobic digestion; there exist, however, so-called dry treatments which generate digestates with a percentage of dry substance also greater than 35%.

With specific reference to nitrogen it is also known:
- that the total quantity of nitrogen contained in a digestate from methanogenic fermentation is practically the same quantity contained in the original biological material, and
- that the total amount of nitrogen consists of an organic part and an inorganic part and
- that the biological materials and in particular the digestates may undergo chemical or biological treatments aimed at hydrolysing the proteins so as to transform the nitrogen from organic to inorganic (mineral form).

In turn the inorganic part is the greatest part formed by ammoniacal nitrogen, but comprises, albeit minimally, nitric nitrogen and nitrous nitrogen.

In detail, the total nitrogen present in the biological materials and in particular in the digestates is on average between 1% and 6%, expressed in relation to the dry substance, and the ammoniacal fraction is on average between 50% and 80% of the total nitrogen if said materials have undergone hydrolytic pre-treatment.

In the case where biological materials and in particular digestates are used over small areas of agricultural land, the presence of nitrogen in large quantities may, however, have negative consequences for the environment, such as pollution of the water tables and eutrophication of sea water; processes for treating said materials have therefore been developed with the aim of reducing the percentage amounts of nitrogen present in them by means of transformation of the organic nitrogen into ammoniacal nitrogen and subsequent extraction (stripping) of the ammonia with the formation of ammonium sulphate and/or other fertilizers. Although performing their function, these processes nevertheless generate a residual digestate which normally has a pH higher than the values considered to be neutral, thus drastically reducing its use on farmland; in addition, the separated nitrogen cannot be easily recycled and is normally used to produce fertilizers with a high nitrogen content for use in agriculture, i.e. in specific geographical areas which are not subject to the obligation to use large quantities of organic manure.

An example of this approach is described in DE 10 2009 020607, which describes a method for purifying an exhaust gas from biogas combustion by means of the production of a nitrogenous fertilizer; this process envisages:
- a first step during which an inorganic base such as calcium hydroxide is added to a starting digestate in order to increase greatly the alkalinity of the effluent digestate which is heated at a low pressure to release the ammonia from it;
- a following step during which the resultant biomass, from which the ammonia has been released, is brought into contact with the exhaust gases resulting from the combustion of biogas and, owing to the acid-base reaction between the nitrogen oxides NOx of the exhaust gas and the alkaline digestate, the elimination of the NOx and the formation of a nitrogenous digestate is achieved.

Considering that the plants for the production of biogas for livestock and agro-industrial activities create a localized concentration of excess amounts of nitrogen which must be managed taking into account both the environmental aspects and the economic aspects, it is often required to transfer these fertilizers with a high nitrogen content from the production area (usually close to the aforementioned activities) to the appropriate disposal areas, said transfer necessarily being performed by means of lorries or tankers with a consequent increase in costs and emissions. Therefore, the problem of production of large quantities of nitrogen, which are concentrated in small geographical areas, is made worse by negative economic aspects - not insignificant in terms of impact - associated with the difficulties of commercial distribution and transportation of the nitrogenous fertilizers produced.

A plant for the production of biogas is often associated with a cogeneration unit, such as a reciprocating gas engine, but also a turbine, which makes use of the biogas produced in order to produce electric and thermal energy.

JP 2009-066559 A illustrates a biogas system designed for the treatment of ammonia produced by the methanogenic digestion/fermentation of biomass; the system envisages the release of ammonia by means of the decarboxylation treatment of the digestate, followed by the injection of air, and a chemical column reactor, which is an oxidation tower, having the sole purpose of eliminating NH3, without using reaction catalysts while monitoring the production of NOx.

The NH₃ is burned inside the chemical reactor by means of the methane produced by digestion, and the oxidation of NH₃ takes place inside an upper zone of the tower, which is divided into two parts which contain metal wire objects or perforated metal plates which may act as catalysts, without energy recovery, nor reduction in the amounts of nitrogen and carbon dioxide produced, but only in order to transform the ammonia, extracted from the digestate, into inert diatomic nitrogen to be released into the atmosphere, with a negative polluting effect on the atmosphere.

The aim of JP H06 343994 A is to remove the phosphorous and the nitrogen from the dehydrated filtrates of digested sludge and to fix the CO₂ levels of exhaust gases from biogas combustion and eliminate the NOx.

It describes how biogas is produced by means of methanogenic digestion of biomass, this biogas being then refined and burned in an oven. The exhaust gases from the latter are mixed with the NH₃ stripped from the dephosphorized spent digestate and are treated in a catalytic reactor in order to eliminate the NOx, which are broken down and reduced inside said reactor.

The gaseous mixture output, devoid of NOx, but containing NH₃ and CO₂, passes into a reactor containing HCl for denitrification, with formation of ammonium chloride; it is then passed into another reactor and bubbled through the denitrified digestate, where it performs fixing of CO₂, by means of absorption cells, using the alkali contained in the digestate to form CaCO3. This consists of an elaborate catalytic converter in which the stripped NH₃ is not used for energy purposes; on the contrary, said NH₃ is regarded as constituting merely a polluting problem on a par with the NOx. The main technical problem which is posed, therefore, is that of developing a process for treating biological materials and/or digestates produced by methanogenic fermentation and with a high nitrogen content, which envisages the extraction of the nitrogen from them so as to overcome the problems described above of managing the nitrogen produced and separated, eliminating the need to use it again in the agricultural chain, generating at the same time organic mixtures suitable for use as correctives for agricultural soils.

In connection with this problem it is also required that the process should result in an improvement in the overall energy balance of the cycle, reducing the quantity of fuel which must be supplied in order to promote the chemical reactions needed for the various steps of the process and allowing energy recovery.

In addition to the above it is also desired that the process should allow the limitation, as far as possible, of the emission into the atmosphere of polluting agents, in particular nitrogen oxides and CO₂, resulting from the process of elimination of the nitrogen from the starting digestate and/or other processes dependent upon biogas plants.

These results are obtained according to the present invention by a process according to the characteristic features of Claim 1 and by a plant according to the characteristic features of Claim 14.

Therefore, according to a first aspect, the invention relates to a process for elimination of nitrogen from biomass and reuse thereof for energy purposes, which comprises the following steps:
A) alkaline conditioning of the biological starting material DIG0, adding calcium oxide (CaO) thereto and forming a first biomass DIG1 with pH>10 and free ammonia (NH₃) in gaseous form;
B) stripping of the ammonia (NH₃) from said first biomass DIG1 resulting from step A) via injection of air, with generation of a second biomass DIG2, free from ammonia, with low nitrogen content and alkaline pH, and a gaseous fluid containing air, ammonia (NH₃) and water vapour;
C) introduction of the gaseous fluid GAS1 resulting from step B) into an internal combustion cogenerator, fuelled by biogas or other fuel, with use of the ammonia (NH₃) contained in the GAS1 for energy purposes, by means of combustion thereof together with the main fuel, and reduction of the nitrogen oxides present in the exhaust gases of the cogenerator by means of catalytic converters;
D) purification, from nitrogen oxides, of the gaseous discharge fluid GAS2 containing NOx, H₂O, CO₂ and N₂ generated during step C) by means of:
   - injection of the gaseous exhaust fluid into the said biomass resulting from step B);
   - combination of the nitrogen oxides (NOx) of the gaseous fluid with the alkaline mass of the biomass with formation of calcium nitrite [Ca(NO₂)₂] and calcium nitrate [Ca(NO₃)₂] and simultaneous lowering of the pH of the biomass;
   - production of a gaseous fluid devoid of NOx and a resultant biomass with pH lower than the pH of the biomass resulting from step B).

Preferably, the step B) of stripping the NH₃ comprises the following steps:
- injection of air into the first biomass, with generation of
- said fluid in the gaseous state, containing air, ammonia (NH₃) and water vapour and
- said second biomass, free from ammonia, with low nitrogen content and high pH.

The injection of air with the generation of bubbles rising up from the bottom of the biomass allows effective stripping of the ammonia which may be drawn off externally in the form of a gaseous fluid containing ammonia NH₃, air and water vapour. Moreover, a second biomass is obtained with a percentage content of nitrogen corresponding to only the quantity of organic nitrogen originally contained in the starting biomass.

In step B it is also preferable to perform condensation of the water vapour present in the gaseous fluid containing ammonia NH₃, air and water vapour; in a combustive mixture of biogas and ammonia, intended for an endogenerator, the presence of water vapour must be limited as far as possible.

Preferably the alkaline condition of the starting biomass is performed by adding calcium oxide (CaO) at a temperature of between 50°C and 70°C and preferably ≥65°C. The addition of calcium oxide causes, in addition to raising of the pH, sanitization of the digestate with elimination of the various pathogenic bacteria contained in it (clostridium, enterobacteriaceae, etc.) and the hydrolysis of the protein residues still present, favouring the mineralization of that nitrogenous fraction.

Advantageously, according to preferred aspects of the invention, it is envisaged that the combustion of the extracted ammonia together with the main fuel takes place inside the cogenerator, while the air contained in the gaseous fluid resulting from stripping is used as part of the combustive agent required, together with air taken from the atmosphere. This allows an increase in the energy efficiency of the cogenerator, by means of the added ammonia, or a reduction, for the same energy efficiency, of the fuel requirement of the said cogenerator.

With the process according to the invention it is possible to process effectively biological starting materials such as: digestates from methanogenic fermentation, livestock manure, biological sewage sludge and residual biomass, but also poultry manure from free-range farms; these biological starting materials may be treated as such or following treatment with procedures for hydrolysing the proteins so as to change the nitrogen from organic into mineral form.

Preferably the process comprises a further step F) of washing with water the gaseous fluid, purified from the NOx, for elimination of the residual NH₃, with formation of a gaseous fluid containing only carbon dioxide (CO₂) and diatomic nitrogen (N₂) and discharge into the atmosphere of said gaseous fluid GAS4. In this way the product discharged into the atmosphere consists of a gas which, in addition to the water vapour, contains only CO₂ and N₂, thus favouring environmental sustainability.

The process according to the invention may envisage a further step E) of neutralization of the pH of the resultant biomass with transformation thereof into an agricultural corrective for agricultural soils. This neutralization step E) is performed by means of addition of sulphuric acid (H₂SO₄) with generation of a mixture of organic substance called defecation gypsum or by means of the addition of carbon dioxide (CO₂) from the exhaust of the cogenerator, with generation of a mixture of organic substance called defecation calcium carbonate. Particularly efficient agricultural correctives are thus produced.

A plant for implementing the process according to the invention is also presented, preferred embodiments of the plant being described in Claims 14-21 and in the following description.

Further details may be obtained from the following description of a non-limiting example of embodiment of the subject of the present invention, provided with reference to the accompanying drawings, in which:

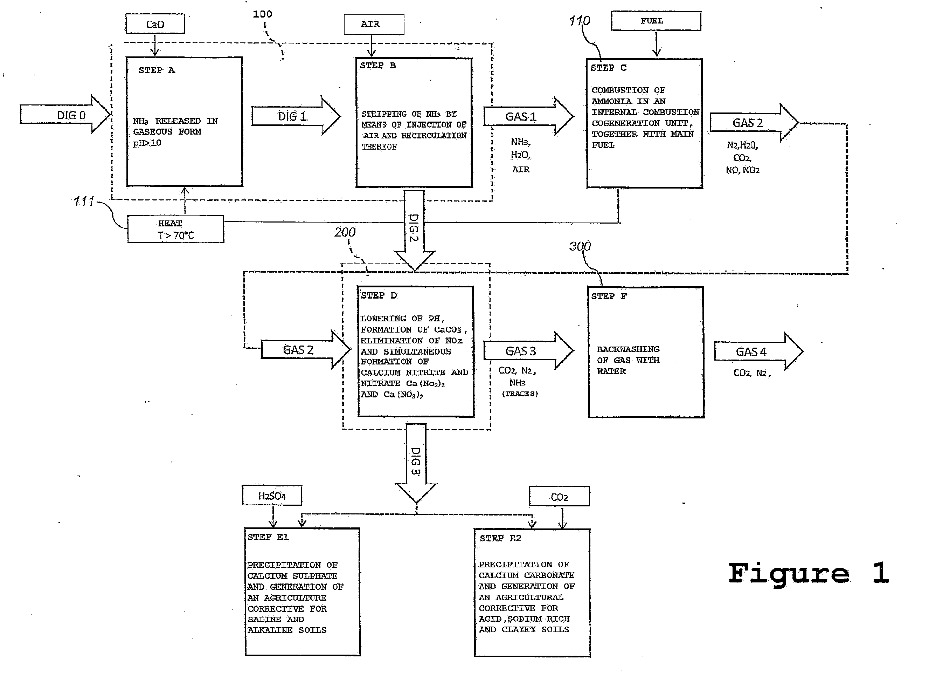

Figure 1: shows a block diagram illustrating the process sequences of the process according to the present invention;

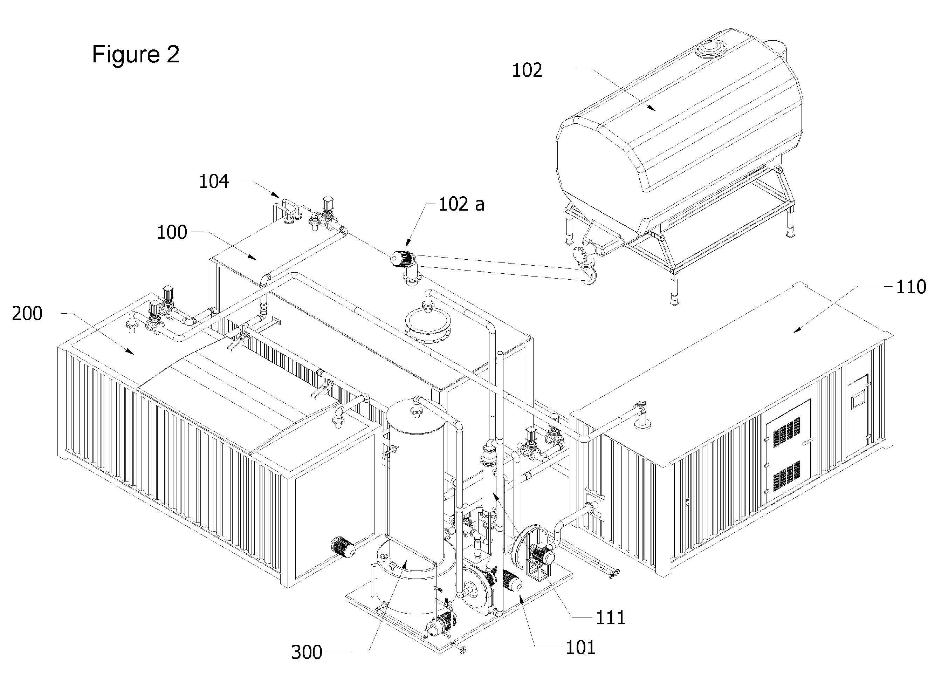

Figure 2: shows a perspective view of an example of a plant for implementing the process according to the present invention.

A process according to the invention for treating biological starting materials DIG0 will now be described with reference to a plant representing a preferred example of embodiment thereof; according to these preferred embodiments it is envisaged that:
**STEP A):** The biological starting material DIG0 is in the fluid state, namely with a dry substance content preferably not greater than 10%, and is conveyed, via the pump 101, inside a first closed reactor 100 which is heated to a temperature greater than or equal to 65 °C, preferably 70°C, which is maintained during the treatment.
   A mixer inside the first reactor 100 keeps the mass thoroughly stirred. Via suitable feeding means 102a a measured amount of live lime (CaO) in powder form, supplied from a silo 102, is introduced until a pH>10 is reached.
   At this point, after being heated to a temperature higher than 70°C and with a pH increased to above 10, the digestate DIG0 is transformed into a biological mass called DIG1 which releases ammoniacal nitrogen in gaseous form.
   The addition of calcium oxide causes an exothermic and oxidative reaction which performs, in addition to raising of the pH, sanitization of the digestate with elimination of the various pathogenic bacteria contained in it (clostridium, enterobacteriaceae, etc.) and hydrolysis of the protein residue still present, favouring the mineralization of that nitrogenous fraction.
   Preferably heating is achieved by means of recovery of the thermal waste of a cogeneration unit, performed by means of a water/water heat exchanger. Preferably the level of the DIG0 is kept at half the total capacity of the reactor 100, so as to create an air channel between the surface of the fluid and the roof of the reactor; the pH of the digestate DIG0 is monitored by a pH meter which controls the means 102a for introducing live lime depending on the pH detected.
**STEP B):** An internal jet pump (not shown) is activated and draws off the air present in the free space above the mass and injects it into the DIG1 at the bottom of the reactor 100, mixing it thoroughly. The air rises back up in the form of bubbles in DIG1, conveying with it the ammonia in gaseous form.
   As mentioned, preferably, the reactor 100 in which DIG1 is situated has a volume about twice that of the digestate contained therein, such that the hot air present, which tends to create an overpressure in the mixer, is drawn off and injected at the bottom of the said reactor 100 so as to remix the mass and free ammonia, this process also normalizing the slight initial overpressure of the reactor 100 resulting from the operating temperature of about 70°C.
   The ammonia is therefore concentrated in the free space of the reactor 100 from where, preferably, following dehumidification via a condenser 111, it is vented and drawn off via a blower and conveyed to the following stage, at the same time as entry of new air into the reactor 100. It is advantageous to perform dehumidification by means of the condenser 111 since, in a combustive mixture of biogas and ammonia, intended for an endogenerator (see the following step C), the presence of water vapour must be limited as far as possible.
   The formation of bubbles rising up from the bottom of the reactor allows stripping of the ammonia which collects in the head space and is then drawn off outside of the reactor 100.
   A fluid gas GAS1 containing ammonia NH₃, air and water vapour, due to heating of the mass, is therefore output from the reactor.
   At the end of stripping, i.e. when it is no longer possible to extract ammonia, the digestate DIG1 is transformed into an alkaline mass DIG2, which has a percentage content of nitrogen corresponding to the sole quantity of organic nitrogen originally contained in the biomass. The alkaline mass DIG2 is pumped into a second reactor 200, preferably with the same dimensions as the first reactor, inside which it is treated as will become clear from the description of the next step D).
**STEP C):** The fluid GAS1, consisting essentially of atmospheric air containing a quantity of ammonia on average of between 5 and 10 g/Nmc, is conveyed to the intake manifold of the cogeneration unit (referred to below also as "cogenerator", "engine" or "endogenerator").
   The air requirement of the engine depends on the number of revolutions and the fuel introduced.
   An automatic valve meters the quantity of atmospheric air entering the engine, in relation to the flow of GAS1 arriving from the stripping system described in the preceding step B).
   The flow of GAS1 (i.e. the stripping flow) will always be less than or equal to the air requirement of the engine and the flowrate will be regulated by jet pump installed in the reactor 100.
   Inside the cogenerator, the GAS1 is mixed with biogas (or other fuel) from the engine components, and the ammonia content, which separates during combustion, releases heat. For the same power output of the cogenerator, the introduction of ammonia allows the saving of a significant amount of biogas (or other fuel).
   In fact, ammonia has a lower calorific value of 4398 kcal/kg compared to 3100-4600 kcal/kg of biogas.
   With reference to a preferred example of the plant for implementing the method described, according to preferred modes of implementation, it is envisaged that the use of ammonia which can be stripped from a digestate resulting from methanogenic fermentation is sufficient to replace volumes of biogas of between 5 and 15% without variation in the production of electrical and thermal energy output from the cogenerator.
   During the combustion phase, two different chemical reactions affect the ammonia.
   During a first reaction (in the presence of carbon derived from the biogas), the separated ammonia generates in flame form HCN which is then converted into NH or NH₂. These react with the oxygen, producing NO + H₂O and the quantity of NO is dependent on the ratio NO/O₂ present in the flame zone.
   This reaction causes an increase in the NOx inside the cylinders during the combustion phase. Simultaneously during a second reaction, which is called selective non-catalyzed reduction of the nitrogen oxides (SNCR), the ammonia, in the presence of oxygen, combines directly with the nitrogen oxide in order to produce diatomic nitrogen and water: 4NO+4NH3+O2 ↔ 4N2+6H2O. This reaction is predominant at temperatures of between 870 and 1200°C. Inside the combustion chamber the temperatures are much higher and the reaction of a direct combination of the ammonia with the oxygen which results in an increase in the NOx becomes competitive. 4NH3+5O2 ↔ 4NO+6H2O.
   An equilibrium is therefore created between the NOx created and those eliminated and depends, not only on the amount of ammonia introduced, but also on the typical temperatures of the engine, on the type of fuel used, on the quantity of air introduced and the type of carburation.
   Even in the presence of an SNCR reaction there is nevertheless an increase in the overall NOx at the exhaust of the cogenerator compared to operation without ammonia.
   The reduction in the NOx occurs by means of treatment of the fumes via the commercial catalytic converter which is normally installed at the engine exhaust.
   This device is commonly able to eliminate more than 90% of the NOx present in the exhaust gases.
   The combusted gases GAS2, output from the cogenerator, are conveyed to the reactor 200 for the following step D) of further purification and use of the carbon dioxide content.
   After extraction of the ammonia, the DIG2 has: a high pH, nitrogen practically consisting of only organic nitrogen, as well as a significant amount of organic substance; the DIG2 may therefore be usefully employed in agriculture for the correction of soils, in particular during pre-ploughing as a basic fertilizer. The provision of lime (introduced for ammonia stripping) may also have beneficial effects when the DIG2 is distributed over acid ground, while the organic substance increases the buffering capacity of the ground, thus balancing the effect of the lime on the pH.
   According to the invention it is also envisaged that the process comprises further steps for purifying, from nitrogen oxides, the gaseous fluid GAS2 containing NOx, H₂O, CO₂ and N₂ resulting from the preceding steps.
   Preferred methods for treating said gaseous fluid GAS2 are described below.
**STEP D)** Purification of the fluid GAS2 by means of bubbling in DIG2. In greater detail the process comprises preferably the following further steps D1) and D2) :
   D1) provision of a second digestate DIG2 with low nitrogen content and pH higher than neutral;
   D2) injection of the gaseous fluid GAS2 into said second digestate DIG2 with low nitrogen content and pH higher than neutral, with production of a gaseous fluid GAS3 free from NOx and a third digestate DIG3 with pH lower than that of the second digestate DIG2.
   According to the invention it is further envisaged that the alkaline mass of the digestate DIG2 is used as a bubbling tank for performing the step of bubbling of the gaseous flow GAS2 which is injected into the bottom of the mass inside a reactor so as to mix with the digestate DIG2;
   the nitrogen oxides [NOx], by passing through the mass, which is alkaline owing to Ca(OH)₂, causes the reaction between the nitrogen oxides NO,NO₂ and the calcium hydroxide Ca(OH)₂, with formation of calcium nitrite [Ca(NO₂)₂] and calcium nitrate [Ca(NO₃)₂] which are both fertilizers; consequently, by passing through alkaline mass, the transformed nitrogen oxides Nox (which are potentially harmful if contained in atmospheric emissions) remain in the third digestate DIG3 resulting from the process and therefore are no longer present in the gaseous output flow GAS3;
   during bubbling the following reactions also occur:
   - the diatomic nitrogen is not retained by the conditions acting on the digestate and proceeds into the resultant gaseous flow GAS3;
   - part of the carbon dioxide, which is present in the gas flow, reacts with the calcium ion Ca²⁺ present in the DIG2 to give CaCO₂;
   - at the same time, the reaction of the carbon dioxide with is combined with the calcium to give calcium carbonate CaCO₃, causes a gradually lowering of the initial pH, favouring any subsequent neutralization of the resultant third digestate DIG3;
   - the percentage of carbon dioxide which has not reacted with the calcium is present in the output flow GAS3.
   Therefore the carbon dioxide contained in GAS2, combines with the calcium ions present in DIG2 to form calcium carbonate salts and performs the function of lowering the pH down to values close to neutral, thus improving the suitability of the resultant digestate DIG3 for agricultural use. This furthermore causes a reduction of the NOx with the calcium ions, which on the one hand results in formation in the digestate DIG3 of nitrites and nitrates useful for agriculture and on the other hand allows in particular a further qualitative improvement of the output flow GAS3 in terms of its polluting content. This therefore results in a significant reduction of the carbon dioxide and NOx content in GAS3, with all the advantages of ecological sustainability of the process.
   Moreover, the injection of air performed in step B) is particularly advantageous as regards the system for recirculating said air inside the reactor 100, since the quantity of air, used and necessary, remains strictly dependent on the intake volume of the cogenerator which must use it up entirely, not being able to operate with too little or too much air.
   In this way there is no excessive use of air taken from the atmosphere since it is exclusively the operating condition of the cogenerator and its technical characteristics which determine the amount required.
   Since there may also be traces of ammonia still present in the gaseous flow GAS3 output from the reactor, it could be preferable to treat the GAS3 before it is discharged into the atmosphere, so that it remains within the statutory limit values for NH₃ emissions.
   According to preferred embodiments of the invention it is further envisaged that the digestate DIG3, resulting from the process of purification of the fluid GAS2 from NOx performed by means of bubbling in the digestate DIG2, is subjected to a further step involving
   E) neutralization of the pH of the DIG3 with transformation thereof into an agricultural corrective for agricultural soils.
      In addition, according to preferred embodiments of the invention, it is envisaged that the GAS3 resulting from the process for NOx purification is subjected to a step involving:
   F) washing with water the fluid GAS3 resulting from step D) for final elimination of atmospheric pollutants, with formation of a fluid GAS4 containing only carbon dioxide CO₂ and diatomic nitrogen N2 which may therefore be discharged into the atmosphere.
      Preferred modes of implementation of these steps E) and F) are described below.
**STEP E)** Neutralization of the digestate DIG3 which has a low content of nitrogen obtained from step E) by means of:
   E1) addition of sulphuric acid H₂SO₄ with generation of a mixture of organic substance called defecation gypsum which can be used as a corrective for saline and/or alkaline agricultural soils; or
   E2) addition of CO₂ with generation of a mixture of organic substance called defecation calcium carbonate which can be used as a corrective for agricultural soils which are acid, rich in sodium or clayey.
      Preferred methods of neutralizing a digestate with a low nitrogen content and high pH are described in the patent application IT-MI2012A 1706 and IT-MI2012A 1707 in the name of the same present Applicant, which are cited herein and to which reference may be made for a complete description of steps E1) and E2). In greater detail step E1) produces a mixture containing an organic substance and calcium sulphate (CaSO₄) - described in the co-pending patent application IT-MI2012A 1706 - which falls within the definition of "defecation gypsum" considered to be the by far most versatile agricultural corrective since it is one of those extremely rare materials which have a beneficial effect in various soil treatment situations.
      The step E2 instead results in the production of the agricultural soil corrective - described in the co-pending patent application IT-MI2012A 1707-which is also regarded as being one of the most complete and neutral correctives for farmland. Although in the aforementioned patent applications processes for the neutralization of digestates with a pH>10 and nitrogen content <0.12% in relation to the dry substance are described, the same processes are also applicable to the biomass resulting from step D) for elimination of the nitrogen oxides from the gaseous fluid GAS2 according to the present invention.
**STEP F)** The gas flow GAS3 obtained in step D) is treated in a water scrubber 300 for eliminating any traces of ammonia which may still be present. The CO₂ which forms part of the flow makes the water acid owing to the carbonic acid which combines with the ammonia generating ammonium carbonate (NH₄)₂CO₃ which has the characteristic of being soluble in water and may therefore accumulate in the recirculating water of the scrubber 300. Once its concentration approaches the solubility limit, the tank may be emptied and in preferred embodiments the resultant solution may be pumped into the reactor 100 as starting digestate DIG0 at the start of a treatment cycle; at temperatures above 60°C, in fact, the ammonium carbonate is broken down into CO2, NH₃ and H₂O.
   At the end of treatment, the emissions discharged into the atmosphere therefore consist of a gas GAS4 which, in addition to water vapour, contains only CO₂ and N₂.

Although the process according to the invention has been described with reference to an example in which the starting biomass has already undergone anaerobic digestion of the fluid type, namely with a dry substance content <10%, it is envisaged that the process and the plant according to the invention are applicable also to the case where:
- the biological starting material DIG0 consists of undigested biomass such as livestock and/or poultry manure, biological sewage sludge and residual biomass and has a dry substance content <10%; in this case the digestate (DIG2), which is ammonia-free, has a pH≥10 and a residual organic nitrogen content on average of between 90% and 60% of the total initial nitrogen.

The process according to the invention is furthermore also applicable, albeit in plants different from that described, to
- a biological starting material DIG0 consisting of poultry manure from free-range farms, characterized by a dry substance content on average of between 45% and 55% and a density of between 0.4 and 0.6.

Said materials may undergo the present process both as such and after having already undergone other chemical or biological treatments for hydrolysing the proteins in order to modify the nitrogen present from organic form to mineral form.

It is therefore clear how the process according to the present invention is able to provide a solution to numerous problems arising from the need to recondition various biological materials such as digestates from methanogenic fermentation, livestock manure, biological sewage sludge and residual biomass, resulting in:
- a reduction in the nitrogen present in the cycle for recovery of biomass for use as fertilizer on farmland, said extracted nitrogen being broken down into its diatomic form which may released into the atmosphere;
- utilization of the nitrogen extracted in the form of ammonia, owing to energy recovery resulting from combustion of the ammonia, extracted from the digestates, as a combustion additive in any cogenerator apparatus;
- a consequent increase in the energy efficiency of the cogenerator, by means of the intake of added ammonia, or a reduction, for the same energy efficiency, of the fuel requirement of the said cogenerator.
- utilization of the exhaust gases produced by the cogenerator which has burned the ammonia together with the biomass, using the carbon dioxide to form calcium carbonate, resulting in neutral adjustment of the pH of the denitrified digestate and fixing of the NOx in the form of salts (nitrites and/or nitrates) which are also useful for agriculture;
- generation of mixtures of organic substances falling within the definition of defecation gypsums and calcium carbonates which can be used for the correction of agricultural soils;
- elimination of the nitrogen oxides from gases containing CO₂, N₂, H₂O, NO and NO₂ which, following treatment, may be released into the atmosphere;
- the execution of an integrated cycle, in which exhaust gases which are decidedly less polluting are released into the atmosphere since they have been significantly purified of carbon dioxide and NOx.

The fertilizers produced in steps E1 and E2 have in fact an extremely beneficial action both on "saline" soils, which are rich in sodium, and on "non-saline alkaline" soils, which are also rich in sodium and/or potassium; in these situations in fact the gypsum favours rebalancing of the cation-exchange capacity (CEC), causing displacement of the monovalent, alkaline, metal ions from the colloidal particles in the ground (sodium and potassium), while favouring the bivalent alkaline ions (calcium and magnesium).

The gypsum directly provides calcium, which is needed by plants for strengthening of the cell walls, so that they are more resistant to diseases and frost, and sulphur is also fundamental for the activity of the useful bacterial flora in the ground, while the calcium carbonate performs beneficial correction of acid soils.

Although described in connection with a number of embodiments and a number of preferred examples of implementation of the invention, it is understood that the scope of protection of the present patent is determined solely by the claims below.

## Claims

1. Process for elimination of nitrogen from biomass DIG0 and reuse thereof for energy purposes, **characterized in that** it comprises the following steps:
A) alkaline conditioning of the biological starting material DIG0, adding calcium oxide (CaO) thereto and forming a first biomass DIG1 with pH>10 and free ammonia (NH₃) in gaseous form;
B) stripping of the ammonia (NH₃) from said first DIG1 biomass resulting from step A) via injection of air, with generation of a second biomass DIG2, free from ammonia, with low nitrogen content and alkaline pH, and a gaseous fluid GAS1 containing air, ammonia (NH₃) and water vapour;
C) introduction of the gaseous fluid GAS1 resulting from step B) into an internal combustion cogenerator fuelled by biogas or other fuel, with use of the ammonia (NH₃) contained in the GAS1 for energy purposes, by means of combustion thereof together with the main fuel, and reduction of the nitrogen oxides present in the exhaust gas (GAS2) of the cogenerator by means of catalytic converters;
D) purification, from nitrogen oxides, of the gaseous fluid GAS2 containing NOx, H₂O, CO₂ and N₂ generated during step C) by means of:
- injection of the gaseous fluid GAS2 into the said biomass DIG2 resulting from step B);
- combination of the nitrogen oxides (NOx) of the gaseous fluid GAS2 with the alkaline mass of the biomass DIG2 with formation of calcium nitrite [Ca(NO₂)₂] and calcium nitrate [Ca(NO₃)₂] and simultaneous lowering of the pH of the biomass DIG2;
- production of a gaseous fluid GAS3 devoid of NOx and a resultant biomass DIG3 with pH lower than the pH of DIG2.

2. Process according to the preceding claim, **characterized in that** the step B) of stripping of the NH₃ comprises the following steps:
- injection of air into the first biomass DIG1, with generation of
- said fluid GAS1, in the gaseous state, containing air, ammonia (NH₃), and water vapour and
- said second biomass DIG2, free from ammonia, with low nitrogen content and high pH.

3. Process according to any one of the preceding claims, **characterized in that** said alkaline conditioning of the starting biomass DIG0 is performed by adding calcium oxide (CaO) at a temperature of between 50°C and 70°C and preferably ≥ 65°C with formation of said first biomass DIG1 with pH>10 and free ammonia in gaseous form.

4. Process according to any one of the preceding claims, **characterized in that** the combustion of the ammonia contained in GAS1 takes place together with the main fuel in the cogenerator, while the air contained in GAS1 is used as part of the necessary combustive agent and the remaining part is composed of air taken from the atmosphere;

5. Process according to any one of the preceding claims, **characterized in that** the starting biomass DIG0 is composed of biological materials such as: digestates from methanogenic fermentation, livestock manure, biological sewage sludge, residual biomass.

6. Process according to any one of the preceding claims, **characterized in that** the starting biomass is composed of poultry manure from free-range farms, with a quantity of dry matter on average of between 45% and 55% and a density of between 0.4 and 0.6.

7. Process according to any one of the preceding claims, **characterized in that** the starting biomass is fed as such or following treatment with procedures for hydrolysing the proteins so as to change the nitrogen from organic form into mineral form.

8. Process according to any one of the preceding claims, **characterized in that** the formation of calcium nitrate [Ca(NO₃)₂] is obtained by the reaction between nitrogen dioxide (NO₂) and calcium hydroxide Ca(OH)₂.

9. Process according to any one of the preceding claims, **characterized in that** the formation of calcium nitrite [Ca(NO₂)₂] is obtained by the reaction between nitrogen oxide NO and calcium hydroxide [Ca(OH)₂].

10. Process according to any one of the preceding claims, **characterized in that** it comprises a further step F) of washing with water the gaseous fluid GAS3, purified from the NOx, for elimination of the residual NH₃, with formation of a gaseous fluid GAS4 containing only carbon dioxide (CO₂) and diatomic nitrogen (N₂) and discharging into the atmosphere of said gaseous fluid GAS4.

11. Process according to any one of the preceding claims, **characterized in that** it comprises a further step E) of pH neutralization of the biomass DIG3 with transformation of the latter into an agricultural corrective for agricultural soils.

12. Process according to claim 11, **characterized in that** said neutralization step (E) is performed by addition of sulphuric acid (H₂SO₄) with generation of a mixture of organic substance called defecation gypsum or by addition of carbon dioxide (CO₂) from the cogenerator exhaust, with generation of a mixture of organic substance called defecation calcium carbonate.

13. Process according to any one of the preceding claims, **characterized in that** the step B involves condensation of the water vapour present in the GAS1.

14. Plant for the extraction of ammoniacal nitrogen in gaseous form from a biological starting material DIG0, reuse thereof for energy purposes and/or the purification, from nitrogen oxides, of a gaseous fluid GAS2 containing NOx, H₂O, CO₂ and N₂, **characterized in that** it comprises:
- a first reactor (100) for alkaline conditioning of the biological starting material DIG0 and formation of an intermediate biomass DIG1 containing free ammonia (NH₃) in gaseous form and a resultant biomass DIG2, with pH higher than neutral, to be extracted from the first reactor (100) and a gaseous fluid GAS1 containing NH₃, H₂O, air; and
- means (101) for loading the biological material DIG0 and means (102a) for introducing calcium oxide (CaO) into said reactor (100); and
- means for injecting air, and
- means (104) for heating the biomass DIG0; and
- an internal combustion cogenerator (110) fuelled by biogas and/or other fuel, with a catalytic converter on the exhaust for reduction of the NOx, which uses as combustive agent at least the air contained in the GAS1, besides any atmospheric air, and, as fuel, the ammonia contained in GAS1, in addition to the biogas and/or other conventional fuel, and outputs a gaseous fluid GAS2 containing N₂, NOx, H₂O and CO₂; and
- means for extracting the fluid GAS1 from the first reactor (100) and feeding the same to the cogenerator,
- a second reactor (200) for treating the gaseous fluid GAS2 containing NOx, H₂O, CO₂ and N₂, and the biomass DIG2, with generation of a gaseous fluid GAS3 containing CO₂, N₂, NH₃ and a biomass with pH which is alkaline, but lower than that of DIG2.

15. Plant according to any one of the preceding claims, **characterized in that** it comprises means (111) for condensation of the water vapour contained in the GAS1.

16. Plant according to any one of the preceding claims, **characterized in that** said first reactor (100) is closed and comprises a mixer for keeping the mass of biological material DIG0 thoroughly stirred.

17. Plant according to any one of the preceding claims, **characterized in that** said first reactor comprises a pH meter which controls the CaO input means (102a).

18. Plant according to any one of the preceding claims, **characterized in that** said means for NH₃ stripping of the first reactor (100) comprise:
- a jet pump for drawing off the air present in the free space of the first reactor (100) and for injecting said air at the bottom of the first reactor (100); and
- venting means for the ammonia extracted; and
- a blower for drawing off and conveying the ammonia extracted to the cogenerator.

19. Plant according to any one of the preceding claims, **characterized in that** said cogenerator is equipped with a valve for introducing air into the intake manifolds, which regulates the flow of atmospheric air and GAS1 depending on the combustive agent required by the engine.

20. Plant according to any one of the preceding claims, **characterized in that** said valve for introuding the air into the intake manifolds communicates with the jet pump placed in the reactor (100) increasing or decreasing the stripping flow and therefore the GAS1 depending on the combustive agent required by the engine.

21. Plant according to any one of the preceding claims, **characterized in that** it comprises means for purification, from nitrogen oxides, of the gaseous fluid GAS3 containing CO₂, N₂ and NH₃, said means comprising:
- at least one water scrubber (300) for washing the gaseous fluid GAS3 and generating a gaseous fluid GAS4 which is ammonia-free and contains N₂ and CO₂.
